# EUROPEAN PATENT SPECIFICATION

(11) **EP 2 358 376 B1**
(45) Date of publication and mention of the grant of the patent: **10.01.2018**
(21) Application number: 09756881.0
(22) Date of filing: 06.11.2009
(51) Int. Cl.: A61K 35/74, A61P 25/14

(54) **EARLY TREATMENT AND PREVENTION OF INCREASED MUSCLE TONICITY**
FRÜHZEITIGE BEHANDLUNG UND PREVENTION VOR ERHÖHTEM MUSKELTONUS
TRAITEMENT ET PRÉVENTION PRÉCOCES D'UNE AUGMENTATION DE LA TONICITÉ MUSCULAIRE

(30) Priority: 20.11.2008 US 116575 P
(43) Date of publication of application: 24.08.2011
(73) Proprietor: ALLERGAN, INC., Irvine, CA 92612 (US)
(72) Inventor: BLUMENFELD, Andrew M., Del Mar California 92014 (US); IRVINE, Ryan A., Los Angeles California 90034 (US)
(74) Representative: Hoffmann Eitle
(86) International application number: PCT/US2009/063487
(87) International publication number: WO 2010/059436

(56) References cited:
- EP-A1- 1 486 214
- WO-A2-00/15245
- US-A1- 2001 012 828
- US-A1- 2002 086 036
- US-A1- 2004 126 827

## Description

### FIELD OF THE INVENTION

The present invention relates to botulinum toxin for use in methods of preventing muscular disorders associated with upper motor neuron lesions.

### BACKGROUND OF THE INVENTION

Upper motor neuron lesions can lead to a multitude of symptoms, one of which includes muscle spasticity. To date, spastic muscles are treated using botulinum toxins once spasticity has developed and is apparent. Botulinum toxin is commonly delivered to the spastic muscles, thereby weakening or paralyzing them. Once the muscles have been weakened or even paralyzed using one of the many commercially available botulinum toxins, the muscles can be re-worked under intense physical therapy.

The anaerobic, gram positive bacterium *Clostridium botulinum* produces a potent polypeptide neurotoxin, botulinum toxin, which causes a neuroparalytic illness in humans and animals referred to as botulism. The spores of *Clostridium botulinum* are found in soil and can grow in improperly sterilized and sealed food containers of home based canneries, which are the cause of many of the cases of botulism. The effects of botulism typically appear 18 to 36 hours after eating the foodstuffs infected with a *Clostridium botulinum* culture or spores. The botulinum toxin can apparently pass unattenuated through the lining of the gut and shows a high affinity for cholinergic motor neurons. Symptoms of detrimental botulinum toxin intoxication can progress from difficulty walking, swallowing, and speaking to paralysis of the respiratory muscles and death.

About 50 picograms of a commercially available botulinum toxin type A (purified neurotoxin complex) has an LD₅₀ in mice (i.e. 1 unit). One unit of BOTOX® (botulinum toxin type A, Allergan, Inc., Irvine, CA) contains about 50 picograms (about 56 attomoles) of botulinum toxin type A complex. One unit (U) of botulinum toxin is defined as the LD₅₀ upon intraperitoneal injection into female Swiss Webster mice weighing 18 to 20 grams each.

Seven generally immunologically distinct botulinum neurotoxins have been characterized, these being, respectively, botulinum neurotoxin serotypes A, B, C₁, D, E, F and G, each of which is distinguished by neutralization with type-specific antibodies. The different serotypes of botulinum toxin vary in the animal species that they affect and in the severity and duration of the paralysis they evoke. For example, it has been determined that botulinum toxin type A is 500 times more potent, as measured by the rate of paralysis produced in the rat, than is botulinum toxin type B. Additionally, botulinum toxin type B has been determined to be non-toxic in primates at a dose of 480 U/kg which is about 12 times the primate LD₅₀ for botulinum toxin type A (Moyer E et al., Botulinum Toxin Type B: Experimental and Clinical Experience, being chapter 6, pages 71-85 of "Therapy With Botulinum Toxin", edited by Jankovic, J. et al. (1994), Marcel Dekker, Inc.).

Regardless of serotype, the molecular mechanism of toxin intoxication appears to be similar and involve at least three steps or stages. An in-depth discussion of these stages, as well as various uses of botulinum toxins, can be found in the background section of many patents, such as, for example, U.S. Patents 6,641,820; 7,255,866 and 7,438,921. The entire toxic activity of botulinum and tetanus toxins is contained in the L chain of the holotoxin; the L chain is a zinc (Zn²⁺) endopeptidase which selectively cleaves proteins essential for recognition and docking of neurotransmitter-containing vesicles with the cytoplasmic surface of the plasma membrane, and fusion of the vesicles with the plasma membrane. Tetanus neurotoxin, botulinum toxin types B, D, F, and G cause degradation of synaptobrevin (also called vesicle-associated membrane protein (VAMP)), a synaptosomal membrane protein. Most of the VAMP present at the cytoplasmic surface of the synaptic vesicle is removed as a result of any one of these cleavage events. Botulinum toxin serotype A and E cleave synaptosomal associate protein 25 (SNAP-25, 25 kDa). Botulinum toxin serotype C₁ was originally thought to cleave syntaxin, but was found to cleave syntaxin and SNAP-25. Each of the botulinum toxins specifically cleaves a different bond, except botulinum toxin type B (and tetanus toxin) which cleave the same bond. Each of these cleavages block the process of vesicle-membrane docking, thereby preventing exocytosis of vesicle content.

Although all the botulinum toxins serotypes apparently inhibit release of the neurotransmitter acetylcholine at the neuromuscular junction, they do so by affecting different neurosecretory proteins and/or cleaving these proteins at different sites. For example, botulinum types A and E both cleave the SNAP-25, but they target different amino acid sequences within this protein. Botulinum toxin types B, D, F and G act on VAMP, with each serotype cleaving the protein at a different site. Finally, botulinum toxin type C₁ has been shown to cleave both syntaxin and SNAP-25. These differences in mechanism of action may affect the relative potency and/or duration of action of the various botulinum toxin serotypes. Apparently, a substrate for a botulinum toxin can be found in a variety of different cell types. See e.g. Biochem J 1;339 (pt 1): 159-65:1999, and Mov Disord, 10(3):376:1995 (pancreatic islet B cells contains at least SNAP-25 and synaptobrevin).

The molecular weight of the neurotoxic component of various botulinum toxins, for all seven of the known botulinum toxin serotypes, is about 150 kD. Interestingly, the botulinum toxins are released by *Clostridial* bacterium as complexes comprising the 150 kD botulinum toxin protein molecule along with associated non-toxin proteins. Thus, the botulinum toxin type A complex can be produced by *Clostridial* bacterium as 900 kD, 500 kD and 300 kD forms. Botulinum toxin types B and C₁ are apparently produced as only a 700 kD or 500 kD complex. Botulinum toxin type D is produced as both 300 kD and 500 kD complexes. Finally, botulinum toxin types E and F are produced as only approximately 300 kD complexes. The complexes (i.e. molecular weight greater than about 150 kD) are believed to contain a non-toxin hemaglutinin proteins and a non-toxin and non-toxic nonhemaglutinin protein. These two non-toxin proteins (which along with the botulinum toxin molecule comprise the relevant neurotoxin complex) may act to provide stability against denaturation to the botulinum toxin molecule and protection against digestive acids when a botulinum toxin is ingested. Additionally, it is possible that the larger (greater than about 150 kD molecular weight) botulinum toxin complexes may result in a slower rate of diffusion of the botulinum toxin away from a site of injection of a botulinum toxin complex.

Botulinum toxin for therapeutic use is obtained by establishing and growing cultures of *Clostridium botulinum* in a fermenter and then harvesting and purifying the fermented mixture in accordance with known procedures. All the botulinum toxin serotypes are initially synthesized as inactive single chain proteins which must be cleaved or nicked by proteases to become neuroactive. The bacterial strains that make botulinum toxin serotypes A and G possess endogenous proteases and serotypes A and G can therefore be recovered from bacterial cultures in predominantly their active form. In contrast, botulinum toxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the botulinum toxin type B serotype only cleave a portion of the toxin produced. The exact proportion of nicked to unnicked molecules depends on the length of incubation and the temperature of the culture. Therefore, a certain percentage of any preparation of, for example, the botulinum toxin type B toxin is likely to be inactive, possibly accounting for the known significantly lower potency of botulinum toxin type B as compared to botulinum toxin type A. The presence of inactive botulinum toxin molecules in a clinical preparation will contribute to the overall protein load of the preparation, which has been linked to increased antigenicity, without contributing to its clinical efficacy.

High quality crystalline botulinum toxin type A can be produced from the Hall A strain of *Clostridium botulinum* with characteristics of ≤3x10⁷ U/mg, an A₂₆₀/A₂₇₈ of less than 0.60 and a distinct pattern of banding on gel electrophoresis. The known Shantz process can be used to obtain crystalline botulinum toxin type A, as set forth in Shantz, E. J., et al. (Properties and use of Botulinum toxin and Other Microbial Neurotoxins in Medicine, Microbiol Rev. 56: 80-99, 1992). Generally, the botulinum toxin type A complex can be isolated and purified from an anaerobic fermentation by cultivating *Clostridium botulinum* type A in a suitable medium. The known process can also be used, upon separation out of the non-toxin proteins, to obtain pure botulinum toxins, such as for example: purified botulinum toxin type A with an approximately 150 kD molecular weight with a specific potency of 1-2x10⁸ LD₅₀ U/mg or greater; purified botulinum toxin type B with an approximately 156 kD molecular weight with a specific potency of 1-2x10⁸ LD₅₀ U/mg or greater, and; purified botulinum toxin type F with an approximately 155 kD molecular weight with a specific potency of 1-2x10⁷ LD₅₀ U/mg or greater.

A commercially available botulinum toxin containing pharmaceutical composition is sold under the trademark BOTOX® (available from Allergan, Inc., of Irvine, Calif.). BOTOX® consists of a purified botulinum toxin type A complex, albumin and sodium chloride packaged in sterile, vacuum-dried form. The botulinum toxin type A is made from a culture of the Hall strain of *Clostridium botulinum* grown in a medium containing N-Z amine and yeast extract. The botulinum toxin type A complex is purified from the culture solution by a series of acid precipitations to a crystalline complex consisting of the active high molecular weight toxin protein and an associated hemagglutinin protein. The crystalline complex is re-dissolved in a solution containing saline and albumin and sterile filtered (0.2 microns) prior to vacuum-drying. The vacuum-dried product is stored in a freezer at or below -5°C. BOTOX® can be reconstituted with sterile, non-preserved saline prior to injection, such as by intradermal, intramuscular or subcutaneous injection, for example. Each vial of BOTOX® contains about 100 units (U) of *Clostridium botulinum* toxin type A purified neurotoxin complex, 0.5 milligrams of human serum albumin and 0.9 milligrams of sodium chloride in a sterile, vacuum-dried form without a preservative.

To reconstitute vacuum-dried BOTOX®, sterile normal saline without a preservative, (0.9% Sodium Chloride Injection) is used by drawing up the proper amount of diluent in the appropriate size syringe. Since BOTOX® may be denatured by bubbling or similar violent agitation, the diluent is gently injected into the vial. For sterility reasons BOTOX® is preferably administered within four hours after the vial is removed from the freezer and reconstituted. During these four hours, reconstituted BOTOX® can be stored in a refrigerator at about 2°C to about 8°C. Reconstituted, refrigerated BOTOX® has been reported to retain its potency for at least about two weeks.

It has been reported that botulinum toxin type A has been used in clinical settings as follows:
(1) about 75-125 units of BOTOX® per intramuscular injection (multiple muscles) to treat cervical dystonia;
(2) 5-10 units of BOTOX® per intramuscular injection to treat glabellar lines (brow furrows) (5 units injected intramuscularly into the procerus muscle and 10 units injected intramuscularly into each corrugator supercilii muscle);
(3) about 30-80 units of BOTOX® to treat constipation by intrasphincter injection of the puborectalis muscle;
(4) about 1-5 units per muscle of intramuscularly injected BOTOX® to treat blepharospasm by injecting the lateral pre-tarsal orbicularis oculi muscle of the upper lid and the lateral pre-tarsal orbicularis oculi of the lower lid;
(5) to treat strabismus, extraocular muscles have been injected intramuscularly with between about 1-5 units of BOTOX®, the amount injected varying based upon both the size of the muscle to be injected and the extent of muscle paralysis desired (i.e. amount of diopter correction desired);
(6) to treat upper limb spasticity following stroke by intramuscular injections of BOTOX® into five different upper limb flexor muscles, as follows:
   (a) flexor digitorum profundus: 7.5 U to 30 U
   (b) flexor digitorum sublimus: 7.5 U to 30 U
   (c) flexor carpi ulnaris: 10 U to 40 U
   (d) flexor carpi radialis: 15 U to 60 U
   (e) biceps brachii: 50 U to 200 U. Each of the five indicated muscles has been injected at the same treatment session, so that the patient receives from 90 U to 360 U of upper limb flexor muscle BOTOX® by intramuscular injection at each treatment session; and
(7) to treat migraine, pericranial injected (injected symmetrically into glabellar, frontalis and temporalis muscles) injection of 25 U of BOTOX® has showed significant benefit as a prophylactic treatment of migraine compared to vehicle as measured by decreased measures of migraine frequency, maximal severity, associated vomiting and acute medication use over the three month period following the 25 U injection.

It is known that botulinum toxin type A can have an efficacy for up to 12 months (European J. Neurology 6 (Supp 4): S111-S1150:1999), and in some circumstances for as long as 27 months, when used to treat glands, such as in the treatment of hyperhydrosis. See e.g. Bushara K., Botulitum toxin and rhinorrhea, Otolaryngol Head Neck Surg 1996;114(3):507, and The Laryngoscope 109:1344-1346:1999. However, the usual duration of an intramuscular injection of BOTOX® is typically about 3 to 4 months.

The success of botulinum toxin type A to treat a variety of clinical conditions has led to interest in other botulinum toxin serotypes. Some examples of commercially available botulinum type A preparations for use in humans are BOTOX® available from Allergan, Inc., of Irvine, Calif., and DYSPORT® available from Beaufour Ipsen, Porton Down, England. A Botulinum toxin type B preparation (MYOBLOC®/NEUROBLOC®) is available from Elan Pharmaceuticals of San Francisco, Calif. Another botulinum type A toxin is available under the trade name Xeomin®, a preparation that contains the about 150 kD neurotoxic component, free of complexing proteins, which is available from Merz Pharmaceuticals of Germany. Additional botulinum toxin preparations for therapeutic use are available from various manufacturers.

A botulinum toxin has also been proposed for or has been used to treat skin wounds (U.S. Pat. No. 6,447,787), various autonomic nerve dysfunctions (U.S. Pat. No. 5,766,605), tension headache, (U.S. Pat. No. 6,458,365), migraine headache pain (U.S. Pat. No. 5,714,468), post-operative pain and visceral pain (U.S. Pat. No. 6,464,986), hair growth and hair retention (U.S. Pat. No. 6,299,893), psoriasis and dermatitis (U.S. Pat. No. 5,670,484), injured muscles (U.S. Pat. No. 6,423,319) various cancers (U.S. Pat. No. 6,139,845), smooth muscle disorders (U.S. Pat. No. 5,437,291), nerve entrapment syndromes (U.S. Published Patent Application 20030224019, filed February 27, 2003), acne (WO 03/011333) and neurogenic inflammation (U.S. Pat. No. 6,063,768). Controlled release toxin implants are known (see e.g. U.S. Pat. Nos. 6,306,423 and 6,312,708) as is transdermal botulinum toxin administration (U.S. Published Patent Application No. 20040009180, Ser. No. 10/194,805 filed July 11, 2002; U.S. Published Patent Application No. 20050175636, Ser. No. 10/675,020 filed September 29, 2003;). Some examples of useful formulations that contain botulinum toxin can be found, for example, in U.S. Published Patent Application Numbers 20020064536 (Ser. No. 10/047,058, filed January 14, 2002), 20030118598 (Ser. No. 10/288,738, filed November 5, 2002), 20080213315 (Ser. No. 12/109,486, filed April 25, 2008), 20060269575 (Ser. No. 11/499,432, filed August 4, 2006) and 20080108570 (Ser. No. 11/932,910, filed October 31, 2007).

It is known that a botulinum toxin can be used to weaken the chewing or biting muscle of the mouth so that self inflicted wounds and resulting ulcers can heal (Payne M., et al, Botulinum toxin as a novel treatment for self mutilation in Lesch-Nyhan syndrome, Ann Neurol September 2002;52(3 Supp 1):S157); permit healing of benign cystic lesions or tumors (Blugerman G., et al., Multiple eccrine hidrocystomas: A new therapeutic option with botulinum toxin, Dermatol Surg May 2003;29(5):557-9); treat anal fissure (Jost W., Ten years' experience with botulinum toxin in anal fissure, Int J Colorectal Dis September 2002;17(5):298-302); and treat certain types of atopic dermatitis (Heckmann M., et al., Botulinum toxin type A injection in the treatment of lichen simplex: An open pilot study, J Am Acad Dermatol April 2002;46(4):617-9).

Additionally, a botulinum toxin has an effect on spastic toes (Suputtitada, A., Local botulinum toxin type A injections in the treatment of spastic toes, Am J Phys Med Rehabil October 2002;81(10):770-5); idiopathic toe walking (Tacks, L., et al., Idiopathic toe walking: Treatment with botulinum toxin A injection, Dev Med Child Neurol 2002;44(Suppl 91):6); and foot dystonia (Rogers J., et al., Injections of botulinum toxin A in foot dystonia, Neurology April 1993;43(4 Suppl 2)).

Tetanus toxin, as well as derivatives (i.e. with a non-native targeting moiety), fragments, hybrids and chimeras thereof can also have therapeutic utility. The tetanus toxin bears many similarities to the botulinum toxins. Thus, both the tetanus toxin and the botulinum toxins are polypeptides made by closely related species of Clostridium (*Clostridium tetani* and *Clostridium botulinum,* respectively). Additionally, both the tetanus toxin and the botulinum toxins are dichain proteins composed of a light chain (molecular weight about 50 kD) covalently bound by a single disulfide bond to a heavy chain (molecular weight about 100 kD). Hence, the molecular weight of tetanus toxin and of each of the seven botulinum toxins (non-complexed) is about 150 kD. Furthermore, for both the tetanus toxin and the botulinum toxins, the light chain bears the domain which exhibits intracellular biological (protease) activity, while the heavy chain comprises the receptor binding (immunogenic) and cell membrane translocational domains.

Further, both the tetanus toxin and the botulinum toxins exhibit a high specific affinity for gangliocide receptors on the surface of presynaptic cholinergic neurons. Receptor mediated endocytosis of tetanus toxin by peripheral cholinergic neurons results in retrograde axonal transport, blocking of the release of inhibitory neurotransmitters from central synapses and a spastic paralysis. Contrarily, receptor mediated endocytosis of botulinum toxin by peripheral cholinergic neurons results in little if any retrograde transport, inhibition of acetylcholine exocytosis from the intoxicated peripheral motor neurons and a flaccid paralysis.

Finally, the tetanus toxin and the botulinum toxins resemble each other in both biosynthesis and molecular architecture. Thus, there is an overall 34% identity between the protein sequences of tetanus toxin and botulinum toxin type A, and a sequence identity as high as 62% for some functional domains. (Binz T. et al., The Complete Sequence of Botulinum Neurotoxin Type A and Comparison with Other Clostridial Neurotoxins, J Biological Chemistry 265(16); 9153-9168:1990).

However, the use of commonly available botulinum toxins and known treatments leave much to be desired. It is common for the spasticity in the affected muscles resulting from an upper motor neuron lesion to involve central nervous system changes that develop over time and commonly lead to maladaptive neuronal plasticity, which is permanent. Current treatments with botulinum toxin are typically delayed until maladaptive neuronal plasticity is clinically apparent and the patient is either physically impaired (e.g. spasticity has already set in), has developed physically unattractive features or both. Although current uses of botulinum toxins to treat clinically apparent spastic muscles and/or muscles displaying maladaptive neuronal plasticity following an upper motor neuron lesion can have dramatically beneficial effects for a patient, methods need to be developed which can treat the affected muscles before the onset of spasticity or the clinical manifestation of maladaptive neuronal plasticity. If such methods were developed, the results would be highly advantageous and life changing for a patient.

Described herein are methods of treatment whereby muscle spasticity can be avoided and/or its development attenuated. This is beneficial to a patient because much of the muscle spasticity encountered following an upper motor neuron lesion is debilitating to various degrees, depending on the severity of the motor neuron lesion. As such, it would be highly advantageous and would be life changing for a patient if methods could be developed wherein spasticity and maladaptive neuronal plasticity can be avoided.

### SUMMARY OF THE INVENTION

Although the present invention is described herein within the wider context of methods for treating maladaptive neuronal plasticity, it is to be understood that the claimed invention does not extend to methods of treatment as such. Rather, the invention relates to botulinum toxin for use in such methods. For instance, the invention relates to botulinum toxin for use in a method of preventing maladaptive neuronal plasticity, comprising the step of administering a therapeutically effective amount of a botulinum toxin to at least a portion of a 1A sensory afferent of at least one muscle prior to the onset of spasticity and/or before maladaptive neuronal plasticity becomes clinically apparent and wherein said administration prevents or attenuates the development of said maladaptive neuronal plasticity. The invention also relates to botulinum toxin for use in a method of preventing maladaptive neuronal plasticity resulting from an occurrence of an upper motor neuron lesion, comprising the step of administering to the patient a therapeutically effective amount of botulinum toxin type A to at least a portion of a 1A sensory afferent of at least one muscle of the upper or lower limb prior to development of maladaptive neuronal plasticity, said therapeutically effective amount being sufficiently low so as to not induce atrophy in said at least one muscle, and said therapeutically effective amount does not substantially affect the Golgi tendons of said at least one muscle, and the botulinum toxin is administered within 6 months of the occurrence of upper motor neuron lesion.

Presently described are methods of preventing maladaptive neuronal plasticity resulting from upper motor neuron lesions by injection of a low dose botulinum toxin into the mid portion or belly of at least one muscle, specifically to the 1A afferents of the intrafusal fibers. The methods focus on modulating the sensory component of the nervous system which leads to the condition. By injecting botulinum toxin at a sufficiently low dose, into the belly of a muscle for example, which does not result in paralysis, atrophy, or even weakness of the muscle, the sensory component of the central nervous system can be modulated and the patient will not develop, or will develop to a lesser degree, spasticity in the treated region and/or maladaptive neuronal plasticity.

Further, in another embodiment, a method is described of preventing maladaptive neuronal plasticity in a patient in need thereof, comprising the step of administering a therapeutically effective amount of a botulinum toxin to at least a portion of a 1A sensory afferent of at least one muscle and wherein the administration prevents the development of the maladaptive neuronal plasticity.

In one embodiment of the method, the maladaptive neuronal plasticity is a result of at least one upper motor neuron lesion which is a result of a condition selected from the group consisting of a stroke, multiple sclerosis, spinal cord lesion, or a combination thereof. An maladaptive neuronal plasticity can result from various types of brain injury, such as, for example, brain trauma.

In one embodiment, the therapeutically effective amount is sufficiently low as to not induce atrophy and/or substantial paralysis in the at least one muscle. In another embodiment, the 1A sensory afferent that is targeted by neurotoxin administration is located within the belly of the at least one muscle. In yet another embodiment, the administration of the botulinum toxin does not substantially affect the Golgi tendons of the at least one muscle.

In one embodiment of the method, the muscle is located on an upper or lower limb. The muscle of the upper limb can be selected from, for example and not limited to, the group consisting of biceps, triceps, deltoids, trapezious, flexor profundus digitorum, extensor digitorum communis, or combinations thereof. The muscle of the lower limb can be, for example and not limited to, selected from the group consisting of tibialis anterior, peroneus longus and brevis, medial and/or lateral gastrocnemius, soleus, adductor magnus, biceps femoris or or combinations thereof.

In another embodiment, a method is described of preventing maladaptive neuronal plasticity resulting from an upper motor neuron lesion in a patient in need thereof, comprising the step of administering a therapeutically effective amount of botulinum toxin type A to at least a portion of a 1A sensory afferent of at least one muscle of the upper or lower limb prior to development of maladaptive neuronal plasticity, the therapeutically effective amount being sufficiently low to not induce atrophy in the at least one muscle, and the therapeutically effective amount does not substantially affect the Golgi tendons of the at least one muscle.

### DETAILED DESCRIPTION OF THE INVENTION

Described herein are methods of preventing maladaptive neuronal plasticity in at least one muscle in a patient who has suffered from an upper motor neuron lesion using low/non-paralytic doses of botulinum toxin. The methods comprise the step of administering a therapeutically effective amount of a botulinum toxin to at least a portion of a 1A sensory afferent, or 1A afferent, of at least one muscle prior to development of spasticity and/or maladaptive neuronal plasticity.

A "therapeutically effective amount" of botulinum toxin as used herein is generally an amount of the toxin that does not result in weakness or paralysis of a muscle or muscles when administered, such as by injection, into the intrafusal fibers found in the belly of the muscle. The therapeutically effective amount is an amount that will not physically harm a patient or substantially cause any significant side effects. Additionally, the therapeutically effective amount of botulinum toxin delivered to a muscle is an amount that does not substantially affect the Golgi tendon of the muscle. The Golgi tendon organs are situated at the junction of the muscle and tendon insertion, and are avoided by administering toxin, such as by injection, to the mid section of the muscle. Electromyography (EMG) can be used to find the motor end plate region with the detection of end plate potentials. These are usually found at the central portions of the muscle. The dosing of botulinum toxin for muscle weakness, such as with botulinum toxin type A (e.g. BOTOX® or DYSPORT®) for example, has been well established for all groups. An exemplary therapeutic dose used in this invention is an amount that does not result in muscle weakness. An exemplary and useful dilution is 2-4 cc of non-preserved physiological saline (0.9%) per 100 units of botulinum toxin (e.g. a botulinum toxin type A, such as BOTOX®, for example). As one example, for biceps utilizing a botulinum toxin type A (e.g. BOTOX®): e.g. muscle weakness can result from administration of about 25 units or more of BOTOX®, a useful sub therapeutic sensory 1A dose is from about 2.5 to about 5 units.

As an example, about 10 percent to about 25 percent of a known lower limit of a dose that would cause muscle weakness can be useful in accordance with the instant invention, but of course, it is to be understood that other useful doses can be utilized in accordance with the teachings herein, on a patient by patent, botulinum toxin formulation, and case-by-case basis. Such determinations, that is, a determination of a useful dose for a particular patient/case, based on that particular patient's presentation, is routine in the prescription of therapeutics in medical arts. For example, it is routine for practitioners, when treating spasmodic muscles, to titrate botulinum toxin doses up to an amount which induces desired paralysis (e.g. "Blepharospasm and hemifacial spasm: A protocol for titration of botulinum toxin dose to the individual patient and for the management of refractory cases". Ortisi E et al. Eye 2006; 20(8):916-922). Thus accordingly, the reverse is easily determined, that is and in accordance with an aspect of the present invention, determination of a therapeutically effective amount of botulinum toxin that is sufficiently low so as to not result in muscle atrophy and/or unwanted paralysis is easily determined.

Patients suffering from upper motor neuron lesions include those who have suffered from a traumatic event such as a stroke, a traumatic brain injury or a traumatic spinal cord injury. In patients suffering from such traumatic events, it is almost axiomatic that the patient will eventually suffer from muscle spasticity and maladaptive neuronal plasticity. It is an objective of the present methods to administer the botulinum toxin to the 1A sensory afferent of at least one muscle before spasticity is apparent and before central nervous system maladaptive neuronal plasticity changes have developed and become clinically apparent.

The botulinum toxin administration is directed to the intrafusal muscle fiber, or muscle spindle, specifically, the 1A sensory afferent of the selected muscle or muscles. Although the causes and mechanisms of muscle spasticity and central nervous system plasticity resulting from upper motor neuron lesions are not well known, and without wishing to be bound by theory, Applicants postulate that a botulinum toxin can be used to induce a depression of 1A sensory afferent conduction/input to the central nervous system and thereby prevent, treat, and/or modulate conditions associated with upper motor neuron lesions. This can result from a reduction in the release of various neuropeptides/neurotransmitters by targeted nerves.

Commonly in a spastic muscle, the gamma motor neuron is activated by cortical centers via spinal cord pathways resulting from an upper motor neuron lesion. This activation results in a shortening of the muscle spindle thereby leading to increased discharges in the 1A afferents and thus in turn producing increased alpha motor neuron output. Increased alpha motor neuron output leads to contraction of the extrafusal fibers of muscles resulting in spasticity and eventual maladaptive neuronal plasticity.

Current methods of treating muscle spasticity involve administering botulinum toxin, at sufficient doses, into the extrafusal muscle fibers or Golgi tendon, to thereby paralyze, weaken and causing atrophy to the muscle or muscles in the general area of treatment.

The present methods, to the contrary, do not substantially paralyze, atrophy or even weaken a muscle in order to prevent, treat, and/or modulate conditions associated with or resulting from upper motor neuron lesions. Rather, the botulinum toxin is delivered, at lower doses, to the intrafusal muscle fiber, or muscle spindle, specifically, the 1A sensory afferent of the affected muscle or muscles, before spasticity is apparent or maladaptive neuronal plasticity is clinically apparent. The goal of the methods is to modulate or substantially terminate the firing of the 1A afferent. Therefore, after an upper motor neuron lesion, when the higher centers of the central nervous system begin to fire the gamma motor neurons, the 1A afferents will not fire. As a result, the alpha motor neurons will not stimulate contraction of the muscle fibers and spasticity of the muscle will not result or will result to a lesser degree.

It is further theorized that the lack of firing of the alpha motor neurons, despite the stimulation of the gamma motor neurons by the higher centers of the central nervous system, will stimulate the central nervous system to re-map neurological pathways to be controlled by a functioning portion of the brain or spinal cord. In fact, there is documented evidence that the higher centers of the central nervous system begin to remap after an upper motor neuron lesion. If the system is able to properly remap, the result can be restored motor function to the affected muscle or muscles with little or no apparent spasticity or maladaptive neuronal plasticity resulting from the upper motor neuron lesion.

It is therefore, according to one aspect, the present methods administer the botulinum toxin before the onset of muscle spasticity or before maladaptive neuronal plasticity is clinically apparent. "Clinically apparent" or "clinical manifestation" as used herein when describing maladaptive neuronal plasticity refers to the situation wherein it can be determined by a physician that maladaptive plasticity has developed and is detrimentally affecting at least one muscle of a patient. The clinical manifestation can be visual, abnormal tonicity, abnormal weakness, atrophy or the like, that is, those manifestations of upper or lower limb spasticity well known in the art.

In one embodiment, the botulinum toxin is administered immediately after the event leading to the upper motor neuron lesion. In another embodiment, the botulinum toxin is administered within 1 day of the traumatic event, or 1 week of the traumatic event, or within 6 months or even within 1 year after the traumatic event. Regardless, it is important that administration of the botulinum toxin occur before the onset of muscle spasticity and maladaptive neuronal plasticity. The maladaptive neuronal plasticity can become clinically manifested in hours, days or even months after the upper motor neuron lesion or traumatic event leading to the symptoms. The onset of muscle spasticity and clinical manifestation of maladaptive neuronal plasticity is unique to each patient and the events leading to the central nervous system effects.

The present methods require administration of botulinum toxin to the 1A afferents of one or more muscles. Methods of administration of the botulinum toxin to a patient can include virtually any method of local neurotoxin administration known to those of ordinary skill in the art. In one embodiment, the botulinum toxin can be intramuscularly injected. In another embodiment, the botulinum toxin can be delivered via a slow release implant to the muscle or muscles of a patient (exemplary implants are described in U.S. Patent Nos. 6,306,423, 6,312,708, exemplary transdermal use of botulinum toxin is discussed in, e.g., U.S. Patent No. 7,384,918 and U.S. Published Patent Application No. 20040009180, filed July 11, 2002). Other methods of local administration of botulinum toxin are known in the art and are considered within the scope of the present disclosure.

Further, the botulinum toxin can be delivered in one or more different compositions. Although one exemplary composition may only contain a single type of neurotoxin, such as botulinum toxin type A, as the active ingredient to suppress 1A afferent firing, other therapeutic compositions may include two or more types of neurotoxins, which may provide enhanced therapeutic effects of the disorders. For example, a composition administered to a patient may include botulinum toxin type A and botulinum toxin type B. Administering a single composition containing two different neurotoxins may permit the effective concentration of each of the neurotoxins to be lower than if a single neurotoxin is administered to the patient while still achieving the desired therapeutic effects.

The botulinum toxin can further be injected into the patient's muscle in one or more locations within the 1A afferents. The pattern of injections, number of injections, injection sites, amount of toxin per injection site, for example, can be determined on a case-by-case basis by physician, as typically known in the medicinal arts relating to the therapeutic use of botulinum toxins for treatment of various neuromuscular conditions.

The botulinum toxins used herein inhibit at least a portion of the 1A afferents of a patient. The suppressive effects provided by the toxin can persists for at least 4 weeks, several months, such as from about 1 month to about 12 months, or from about 1 month to about 6 months. In one embodiment, the suppression can last for years, for example up to about 2 years.

Exemplary, commercially available, botulinum toxin containing compositions include, but are not limited to, BOTOX® (Botulinum toxin type A neurotoxin complex with human serum albumin and sodium chloride) available from Allergan, Inc., of Irvine, California in 100 unit vials as a lyophilized powder to be reconstituted with 0.9% sodium chloride before use); DYSPORT® (Clostridium botulinum type A toxin haemagglutinin complex with human serum albumin and lactose in the formulation), available from Ipsen Limited, Berkshire, U.K. as a powder to be reconstituted with 0.9% sodium chloride before use) which can be used at about 3 to about 4 times the amounts of BOTOX® as set forth herein in each instance (that is, an amount that does not induce paralysis or muscle atrophy, i.e. the therapeutically useful amount readily determined by one of ordinary skill in the art for use in accordance with the teachings presented herein); and MYOBLOC® (an injectable solution comprising botulinum toxin type B, human serum albumin, sodium succinate, and sodium chloride at about pH 5.6, available from Solstice Neurosciences, Inc., South San Francisco, California) which can be used at about 30 to about 50 times the amounts of BOTOX® as set forth herein in each instance, as known in the art. XEOMIN® (a 150 kDa botulinum toxin type A formulation available from Merz Pharmaceuticals, Germany) is another useful neurotoxin (comprising the neurotoxic component) which can be used at about 1 to about 2 times the amounts of BOTOX® as set forth herein in each instance.

The amount of toxin administered according to a method within the scope of the present disclosure can vary according to the particular characteristics of upper motor neuron lesion induced condition being treated, prevented or modulated, including its severity and other various patient variables including muscle or muscles being injected (number and mass), size, weight, age, and responsiveness of the particular patent to the botulinum neurotoxin therapy. Generally, however, the present methods require low doses of botulinum toxin that do not result in muscle weakness, atrophy or paralysis. That amount is determined on a case-by-case basis. As a general guide the practitioner, typically, no less than about 1 units and no more than about 500 units of a botulinum toxin type A (such as BOTOX®) is administered per injection site (i.e. to each 1A afferent), per patient treatment session (e.g. and in one embodiment, by injection, about 5 units of botulinum toxin type A, such as commercially obtainable as BOTOX®, or about 20 units of DYSPORT®, another commercially available botulinum toxin type A composition, or about 200 units of MYOBLOC®, a commercially available botulinum toxin type B preparation for example, to upper limb muscle such as a biceps muscle, more or less toxin being utilized based on the mass of the muscle to be treated and the particular patient/case). For topical applications, more neurotoxin can be used. For a botulinum toxin type A such as DYSPORT®, preferably no less than about 1 unit and no more about 2000 units of the botulinum toxin type A are administered per administration or injection site, per patent treatment session. For a botulinum toxin type B such as MYOBLOC®, preferably no less than about 1 unit and no more about 20,000 units of the botulinum toxin type B are administered per administer or injection site, per patent treatment session. Less than about 1 unit (of BOTOX®, DYSPORT® and MYOBLOC® respectively) may fail to achieve a desired therapeutic effect, while more than about 500, 2000 or 25000 units (of BOTOX®, DYSPORT® and MYOBLOC® respectively) may result in clinically observable unwanted effects (e.g. muscle paralysis) which can vary depending on administration method, site and particular patient. For example and in particular embodiments, an implant that slowly releases a therapeutically effective amount of botulinum toxin can contain an amount of toxin (i.e. of units) that may be higher than an amount that is typically administered directly (e.g., by intramuscular injection to the belly of the muscle that does result in weakness, atrophy or the like). As an illustrative example, while 100 units of BOTOX® may not be desired to be administered at one time to a 1A afferent of a muscle via a syringe, yet this same 100 units (or even 500 units or whatever desired amount, for example), when incorporated into a slow-release implant that is placed intramuscularly near the 1A afferent, can as such now provide slow, long term dosing/release of low doses of botulinum neurotoxin in therapeutically effective amounts in accordance with the present invention.

In additional embodiments, no less than about 1 unit and no more about 400 units of BOTOX®; no less than about 1 unit and no more than about 1600 units of DYSPORT®, and; no less than about 1 unit and no more than about 20000 units of MYOBLOC® are administered per site, per patent treatment session.

In still further embodiments, no less than about .5 unit and no more about 10 units of BOTOX®; no less than about 1 unit and no more than about 40 units of DYSPORT®, and; no less than about 1 unit and no more than about 500 units of MYOBLOC® are administered per site (e.g., per muscle), per patent treatment session. There can be multiple injection sites (i.e. a pattern of injections or pattern of muscles) for each patient treatment session in order to distribute the neurotoxin over a desired target area or desired set of muscles, such as extensor muscles or flexor muscles, for example. It is to be understood that, and in accordance with the present invention, the amount of botulinum toxin utilized is to be determined on a case-by-case basis (as is the case with the administration of any therapeutic). For example, while a dose of 50 units of a botulinum toxin type A, all to one small muscle of the hand, may result in unwanted muscle paralysis (and achieve a result that is contrary to the teachings herein) of that one small hand muscle, that same 50 unit dose, as understood by one of ordinary skill in the art (taking what is known about botulinum toxin dosing over the past at least 20 years and the instant disclosure at hand) can distribute this 50 units to, for example, 5 to 10 muscles, for example, and thus administer 50 units of botulinum toxin (or any useful amount of botulinum toxin in accordance with the instant disclosure) without inducing unwanted paralysis or muscle atrophy, by simply distributing the toxin as best seen fit for a particular patient's presentation (amount/per muscle(s)).

Although examples of routes of administration and dosages are provided, the appropriate route of administration and dosage are generally determined on a case-by-case basis by the attending physician, as known in the botulinum toxin arts, and titration of the dosage to a therapeutically effective one, for a particular patient/condition, is routinely undertaken. Such determinations are routine to one of ordinary skill in the art (see for example, Harrison's Principles of Internal Medicine (1998), edited by Anthony Fauci et al., 14th edition, and published by McGraw Hill). For example, the route and dosage for administration of a Clostridial neurotoxin, or more specifically a botulinum toxin, according to the present disclosed invention can be selected based upon criteria such as the solubility characteristics of the neurotoxin chosen as well as the intensity and scope of the upper motor neuron lesion.

Additionally, in some embodiments, a physician may have to alter dosage in each case (i.e. patient) in accordance with the assessment of the severity of the condition, as typically done when treating patients with a condition/disorder. Further, in some embodiments, the treatment may have to be repeated at least one additional time, in some cases several times, depending on the severity of the condition and the patient's overall health. If, for example, a patient is not deemed physically suitable for a full administration of botulinum toxin, or if a full administration is not desired for any reason, smaller doses on multiple occasions may prove to be efficacious. If unwanted muscle paralysis is observed for a particular dosage, the amount of botulinum toxin administered can be reduced to avoid muscle paralysis and thus the treatment dose appropriately titrated in accordance with the methods/use as disclosed herein.

The methods described herein require that a botulinum toxin be administered prior to the onset of spasticity and/or before maladaptive neuronal plasticity becomes clinically apparent. Therefore, the botulinum toxin should be delivered to the patient before the onset of spasticity and/or before maladaptive neuronal plasticity is clinically apparent. The time of delivery can be measured from the time of the upper motor neuron lesion, for example within 1 year of a lesion, preferably within 6 months.

The botulinum toxins according to the methods herein are administered specifically to 1A afferents found on and within intrafusal fibers. Intrafusal fibers are found within skeletal and smooth muscles. The administration of botulinum toxin to 1A afferents, as described above, is thought to modulate a sensory component of the central nervous system. Further, administration of botulinum toxin to extrafusal fibers, muscle spindle or the Golgi tendons would have an inhibitory affect on the modulation of the central nervous system component. Therefore, the present methods specifically avoid administration of botulinum toxin into such areas as extrafusal fibers, muscle spindle and the Golgi tendon.

The administration of botulinum toxin to 1A afferents of muscles can include one or more muscles or muscle groups to which administration is appropriate. The muscle or muscles to be treated can be, for example and not limited to, selected from the group consisting of splenius capitis, sternocleidomastoid, scalene complex, levator scapulae, semispinalis, longissimus capitis, longissimus cervicis, multifidus, obliqus capitis inferior, obliqus capitis superior, rectus capitis posterior major, rectus capitis posterior minor, trapezius/pars horizontalis, trapezius/pars cervicalis, suprahyoidal muscles, infrahyoidal muscles, digastricus, pterygoideus medialis, pterygoideus lateralis, masseter, temporalis, orbicularis oculi, nasalis, procerus, corrugator supercilii, depressor anguli oris, depressor labii inferioris, frontalis, levator labii superioris, levator labii superioris alaeque nasi, orbicularis oris, risorius, zygomaticusminor, zygomaticus major, deltoideus, triceps brachii, brachioradialis, biceps brachii, pronator quadratus pronator teres, flexor carpi radialis, flexor carpi ulnaris, flexor pollicis longus, opponens interossei, lumbricales, adductor pollicis, flexor pollicis brevis, flexor digitorum superficialis, flexor digitorum sublimus flexor digitorum profundus, adductor group, quadriceps femoris, , triceps surae, tibialis posterior, flexor hallucis longus, tibialis anterior, extensor hallucis longus, extensor digitorum longus, flexor hallucis brevis, flexor digitorum brevis, and paraverterbal muscles, for example.

Two classes of muscles that can be treated according to the present methods include extensor muscles and flexor muscles. An extensor muscle is any muscle that opens a joint increasing the angle between components of a limb. Alternatively, a flexor muscle is a muscle whose contraction bends a joint, decreasing the angle between components of a limb. In some embodiments, it is useful to inject a particular set of extensor and flexor muscles for a particular limb or joint. Further, in some embodiments, it is advantageous to inject a flexor muscle and not an extensor muscle or vice versa. In certain muscle groups, there can be more than one extensor or flexor relative to a particular joint, and in such cases, at least one of the muscles should be treated according to the methods described herein.

Some exemplary extensor and flexor muscle sets that may be treated according to the methods described herein include, but are not limited to, upper arm muscles including biceps brachii and triceps brachii, upper leg such as the quadriceps femoris; lower leg such as the biceps femoris, semitendinosis, tibialis anterior, gastrocnmemius and soleus, among others, for example.

Further, the muscles of the hands and wrist may be treated according to the present methods. Some exemplary muscles of the hands treated according to the present methods include, but are not limited to, adductor pollicis, flexor pollicis brevis, flexor digitorum superficialis, and flexor digitorum profundus. Any combination of muscles, including those not called out here, can be useful in preventing spasticity within the scope of the present description.

In a further embodiment, the muscles of the face and neck can be treated according to the present methods. In one embodiment, the masseter muscle responsible for contracting the jaw bone can be treated according to the methods described herein.

### Example 1

### Spinal Cord Injury

A 52 year old male weighing 100 kg in stable condition presents at the intensive care unit at a local hospital after a severe car accident leaving him with a severe lesion to the neck disrupting the motor activity from the brain to the upper extremities. It has been 24 hours since the man was cleared from emergency surgery immediately following the accident. Upon examination, a neurologist and a physical therapist, determine that based on the spinal cord injury, the male is at high risk for spasticity in both upper arms due to the disruption of upper motor neuron signal transfer. In order to prevent the impending onset of spasticity in the upper extremities, a low 5 unit dose of botulinum toxin type A (e.g. BOTOX®) is injected into each bicep brachii muscle, specifically targeting the 1A afferents within the belly of the muscle. In order to prevent further weakness in the patient, care is taken so that the botulinum toxin is not injected adjacent (as an example, at least about 1 inch away from) the extrafusal fibers or the Golgi tendons.

After six months of intensive treatment and rehabilitation, the patient does not developed spasticity in either upper extremity. A maintenance dose of botulinum toxin (2.5 units) is administered into the bicep brachii muscles each 6 months to prevent future onset of spasticity in the upper arms as a result of the spinal cord injury. There is no clinical weakness of the biceps present after administration.

### Example 2

### Post Stroke Treatment

A 72 year old female rests comfortably in the intensive care unit of a local hospital after a severe stroke to the left brain. A neurologist and a physical therapist determine that based on the severity and location of the stroke, the female is at risk for spasticity and eventual plasticity on the right side of the body. This eventual spasticity will likely affect the upper extremity flexor muscle group and the lower extremity extensor muscle group.

As a preventative treatment, 12 hours post stroke, the 1A afferents of the major muscles on the right side of the body are treated with low doses of botulinum toxin type A by injection into the center belly of the muscles. The right hand is treated with 1.5 units (e.g BOTOX®) per injection to the adductor pollicis, flexor pollicis brevis, flexor digitorum superficialis, and flexor digitorum profundus. Further, the right side is also treated with 5 units injected to each of the bicep brachii, quadricep femoris, medial and lateral gastrocnemius and soleus muscles.

After six months of intensive treatment and rehabilitation, the patient has not developed spasticity anywhere in her right side. A maintenance dose of 2.5 units of botulinum toxin type A is administered into each treated muscle every 6 months to prevent the onset of spasticity.

### Example 3

### Post Stroke Treatment of the Hands

A 80 year old male rests comfortably in the intensive care unit of a local hospital after a severe stroke to the right brain. A neurologist and a physical therapist, determine that based on the severity and location of the stroke, the male is at risk for spasticity in both hands. Spasticity of the left and right hands would leave the male unable to function normally because he would loose function of both hands.

As a preventative treatment, 1 month post stroke, the 1A afferents of the major muscles in the right and left hands are treated with low doses of botulinum toxin type A. The right and left hands are treated with 1.5 units per injection (e.g. BOTOX®) to the adductor pollicis, flexor pollicis brevis, flexor digitorum superficialis, and flexor digitorum profundus.

After six months of intensive treatment and rehabilitation, the patient has not developed spasticity anywhere in his hands. A maintenance dose of 0.5 units of botulinum toxin to each of the above muscle bellies is administered every 6 months to prevent the onset of spasticity.

While exemplary ranges of a particularly botulinum toxin (here a botulinum neurotoxin type A, e.g. such as BOTOX®)) are specifically called out in the Examples above, other botulinum toxin types may also be utilized in accordance with the teachings of the present invention, as those of ordinary skill in the art will clearly appreciate.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

## Claims

1. Botulinum toxin for use in a method of preventing maladaptive neuronal plasticity, comprising the step of administering a therapeutically effective amount of a botulinum toxin to at least a portion of a 1A sensory afferent of at least one muscle prior to the onset of spasticity and/or before maladaptive neuronal plasticity becomes clinically apparent and wherein said administration prevents or attenuates the development of said maladaptive neuronal plasticity.

2. Botulinum toxin for use in the method according to claim 1, wherein said therapeutically effective amount is sufficiently low to not induce atrophy in said at least one muscle.

3. Botulinum toxin for use in the method according to claim 1, wherein said maladaptive neuronal plasticity is a result of at least one upper motor neuron lesion.

4. Botulinum toxin for use in the method according to claim 3, wherein said upper motor neuron lesion is a result of a condition selected from the group consisting of a stroke, multiple sclerosis, spinal cord lesion, or a combination thereof.

5. Botulinum toxin for use in the method according to claim 1, wherein said 1A sensory afferent is located within the belly of said at least one muscle.

6. Botulinum toxin for use in the method according to claim 1, wherein said administration of said botulinum toxin does not substantially affect the Golgi tendons of said at least one muscle.

7. Botulinum toxin for use in the method according to claim 1, wherein said muscle is located on an upper or lower limb.

8. Botulinum toxin for use in a method of preventing maladaptive neuronal plasticity resulting from an occurrence of an upper motor neuron lesion, comprising the step of administering to the patient a therapeutically effective amount of botulinum toxin type A to at least a portion of a 1A sensory afferent of at least one muscle of the upper or lower limb prior to development of maladaptive neuronal plasticity, said therapeutically effective amount being sufficiently low so as to not induce atrophy in said at least one muscle, and said therapeutically effective amount does not substantially affect the Golgi tendons of said at least one muscle, and the botulinum toxin is administered within 6 months of the occurrence of upper motor neuron lesion.

9. Botulinum toxin for use in the method according to claim 1 or 8, wherein said muscle located on said upper limb is selected from the group consisting of biceps, triceps, deltoids, trapezious, flexor digitorum profundus, extensor digitorum communis, or combinations thereof.

10. Botulinum toxin for use in the method according to claim 1 or 8, wherein said muscle located on said lower limb is selected from the group consisting of tibialis anterior, calf muscle, thigh muscle, or combinations thereof.

11. Botulinum toxin for use in the method according to claim 1 or 8, wherein the botulinum toxin is administered immediately after the event leading to the upper motor neuron lesion.

12. Botulinum toxin for use in the method according to claim 1 or 8, wherein the botulinum toxin is administered within 1 week, preferably within 1 day after the event leading to the upper motor neuron lesion.

## Patentansprüche

1. Botulinumtoxin zur Verwendung bei einem Verfahren zur Prävention maladaptiver neuronaler Plastizität, umfassend den Schritt der Verabreichung einer therapeutisch wirksamen Menge eines Botulinumtoxins an wenigstens einen Abschnitt eines 1A-sensorischen Afferenten wenigstens eines Muskels vor dem Einsetzen von Spastizität und/oder bevor maladaptive neuronale Plastizität klinisch auffällig wird und wobei die Verabreichung die Entwicklung dieser maladaptiven neuronalen Plastizität verhindert oder lindert.

2. Botulinumtoxin zur Verwendung bei dem Verfahren gemäß Anspruch 1, wobei die therapeutisch wirksame Menge ausreichend niedrig ist, so dass keine Atrophie in dem wenigstens einen Muskel induziert wird.

3. Botulinumtoxin zur Verwendung bei dem Verfahren gemäß Anspruch 1, wobei die maladaptive neuronale Plastizität ein Ergebnis wenigstens einer oberen motorischen Neuronenläsion ist.

4. Botulinumtoxin zur Verwendung bei dem Verfahren gemäß Anspruch 3, wobei die obere motorische Neuronenläsion ein Ergebnis eines Zustands ist, ausgewählt aus der Gruppe bestehend aus einem Schlaganfall, multipler Sklerose, Rückenmarkläsion oder einer Kombination davon.

5. Botulinumtoxin zur Verwendung bei dem Verfahren gemäß Anspruch 1, wobei der 1A-sensorische Afferent innerhalb des Muskelbauchs des wenigstens einen Muskels lokalisiert ist.

6. Botulinumtoxin zur Verwendung bei dem Verfahren gemäß Anspruch 1, wobei die Verabreichung des Botulinumtoxins die Golgi-Sehnen des wenigstens einen Muskels im Wesentlichen nicht beeinträchtigt.

7. Botulinumtoxin zur Verwendung bei dem Verfahren gemäß Anspruch 1, wobei der Muskel auf einer oberen oder unteren Extremität lokalisiert ist.

8. Botulinumtoxin zur Verwendung bei einem Verfahren zur Prävention maladaptiver neuronaler Plastizität, die aus einem Auftreten einer oberen motorischen Neuronenläsion resultiert, umfassend den Schritt der Verabreichung an den Patienten einer therapeutisch wirksamen Menge an Botulinumtoxin Typ A an wenigstens einen Abschnitt eines 1A-sensorischen Afferenten wenigstens eines Muskels der oberen oder unteren Extremität vor Entwicklung maladaptiver neuronaler Plastizität, wobei die therapeutisch wirksame Menge ausreichend niedrig ist, so dass keine Atrophie in dem wenigstens einen Muskel induziert wird und die therapeutisch wirksame Menge die Golgi-Sehnen des wenigstens einen Muskels nicht beeinträchtigt und das Botulinumtoxin innerhalb von 6 Monaten ab dem Auftreten der oberen motorischen Neuronenläsion verabreicht wird.

9. Botulinumtoxin zur Verwendung bei dem Verfahren gemäß Anspruch 1 oder 8, wobei der auf der oberen Extremität lokalisierte Muskel ausgewählt ist aus der Gruppe, bestehend aus Bizeps, Trizeps, Deltoiden, Trapezius, Flexor digitorum profundus, Extensor digitorum communis oder Kombinationen davon.

10. Botulinumtoxin zur Verwendung bei dem Verfahren gemäß Anspruch 1 oder 8, wobei der auf der unteren Extremität lokalisierte Muskel ausgewählt ist aus der Gruppe, bestehend aus Tibialis anterior, Wadenmuskel, Oberschenkelmuskel oder Kombinationen davon.

11. Botulinumtoxin zur Verwendung bei dem Verfahren gemäß Anspruch 1 oder 8, wobei das Botulinumtoxin unmittelbar nach dem zur oberen motorischen Neuronenläsion führenden Ereignis verabreicht wird.

12. Botulinumtoxin zur Verwendung bei dem Verfahren gemäß Anspruch 1 oder 8, wobei das Botulinumtoxin innerhalb einer Woche, vorzugsweise innerhalb eines Tages, nach dem zur oberen motorischen Neuronenläsion führenden Ereignis verabreicht wird.

## Revendications

1. Toxine botulique pour une utilisation dans un procédé de prévention de la plasticité neuronale inadaptée, comprenant l'étape d'administration d'une quantité efficace d'un point de vue thérapeutique d'une toxine botulique à au moins une portion d'un nerf sensoriel afférent 1A d'au moins un muscle avant le début d'une spasticité et/ou avant qu'une plasticité neuronale inadaptée devienne cliniquement apparente et dans laquelle ladite administration prévient ou atténue le développement de ladite plasticité neuronale inadaptée.

2. Toxine botulique pour une utilisation dans le procédé selon la revendication 1, dans laquelle ladite quantité efficace d'un point de vue thérapeutique est suffisamment faible pour ne pas induire d'atrophie dans ledit au moins un muscle.

3. Toxine botulique pour une utilisation dans le procédé selon la revendication 1, dans laquelle ladite plasticité neuronale inadaptée est un résultat d'au moins une lésion des motoneurones supérieurs.

4. Toxine botulique pour une utilisation dans le procédé selon la revendication 3, dans laquelle ladite lésion des motoneurones supérieures est un résultat d'un état sélectionné dans le groupe constitué d'un accident vasculaire cérébral, de la sclérose en plaques, d'une lésion de la moelle épinière, ou d'une combinaison de ceux-ci.

5. Toxine botulique pour une utilisation dans le procédé selon la revendication 1, dans laquelle ledit nerf sensoriel afférent 1A est localisé à l'intérieur du ventre dudit au moins un muscle.

6. Toxine botulique pour une utilisation dans le procédé selon la revendication 1, dans laquelle ladite administration de ladite toxine botulique n'affecte sensiblement pas les tendons de Golgi dudit au moins un muscle.

7. Toxine botulique pour une utilisation dans le procédé selon la revendication 1, dans laquelle ledit muscle est localisé sur un membre supérieur ou inférieur.

8. Toxine botulique pour une utilisation dans un procédé de prévention de la plasticité neuronale inadaptée résultant d'une apparition d'une lésion des motoneurones supérieurs, comprenant l'étape d'administration au patient d'une quantité efficace d'un point de vue thérapeutique de toxine botulique de type A à au moins une portion d'un nerf sensoriel afférent 1A d'au moins un muscle du membre supérieur ou inférieur avant le développement de la plasticité neuronale inadaptée, ladite quantité efficace d'un point de vue thérapeutique étant suffisamment faible pour ne pas induire d'atrophie dans ledit au moins un muscle, et ladite quantité efficace d'un point de vue thérapeutique n'affecte sensiblement pas les tendons de Golgi dudit au moins un muscle, et la toxine botulique est administrée dans les 6 mois de l'apparition de la lésion des motoneurones supérieurs.

9. Toxine botulique pour une utilisation dans le procédé selon la revendication 1 ou 8, dans laquelle ledit muscle localisé sur ledit membre supérieur est sélectionné dans le groupe constitué du biceps, du triceps, des deltoïdes, du trapèze, du muscle fléchisseur profond des doigts, du muscle extenseur des doigts, ou de combinaisons de ceux-ci.

10. Toxine botulique pour une utilisation dans le procédé selon la revendication 1 ou 8, dans laquelle ledit muscle localisé sur ledit membre inférieur est sélectionné dans le groupe constitué du muscle tibial antérieur, du muscle du mollet, du muscle de la cuisse, ou de combinaisons de ceux-ci.

11. Toxine botulique pour une utilisation dans le procédé selon la revendication 1 ou 8, dans laquelle la toxine botulique est administrée immédiatement après l'événement menant à la lésion des motoneurones supérieurs.

12. Toxine botulique pour une utilisation dans le procédé selon la revendication 1 ou 8, dans laquelle la toxine botulique est administrée dans la semaine, préférablement dans le jour suivant l'événement menant à la lésion des motoneurones supérieurs.
